# EUROPEAN PATENT APPLICATION

(11) **EP 2 000 468 A1**
(43) Date of publication of application: **10.12.2008**
(21) Application number: 08008778.6
(22) Date of filing: 09.05.2008
(51) Int. Cl.: C07D 401/12, A61K 31/4439, A61P 1/04

(54) **Esomeprazole salts and processes for preparation thereof**

(30) Priority: 09.05.2007 IN ch09882007; 30.08.2007 US 968976 P
(71) Applicant: Dr. Reddy's Laboratories Ltd., Hyderabad 500 016 (IN); Dr. Reddy's Laboratories Inc., Bridgewater, NJ 08807-2862 (US)
(72) Inventor: Bolugoddu, Vijayabhaskar, Sumithra Nagar Kukatpally, Hyderabad 500 027 (IN); Mamilla, Srinivas Reddy, Raja rajeshwari colony, Gorinagar Secundrabad (IN); Prasad Reddy Seerapu, Siva, Visakapatnam (IN); Venkata Annapurma Cheemalapati, Sasikala, Visakapatnam (IN)
(74) Representative: Müller-Boré & Partner Patentanwälte

(57) **Abstract**

There is provided salts of esomeprazole and processes for preparing it. Also provided is a polymorphic form of esomeprazole salts and process for preparing thereof.

## Description

### TECHNICAL FIELD

The present application relates to the esomeprazole salts and processes for their preparation. The present application also relates to polymorphic forms of esomeprazole salts and processes for their preparation.

### BACKGROUND

Esomeprazole has a chemical name (S)-5-methoxy-2-[[(4-methoxy-3, 5-dimethyl-2-pyridinyl)-ethyl] sulfinyl]-1H-benzimidazole (hereinafter referred to by the adopted name "esomeprazole"), is the S-enantiomer of omeprazole, and has the structure of Formula I.

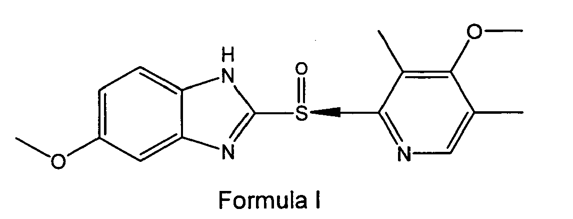

Esomeprazole is a proton pump inhibitor developed as an oral treatment for peptic ulcer, gastroesophangeal reflux disease (GERD), duodenal ulcer and esophagitis.
Esomeprazole magnesium is available in the market under the brand name NEXIUM™ as delayed-release capsules for oral administration. Each delayed release capsule contains 20 mg or 40 mg esomeprazole (present as 22.3 mg or 44.5 mg of esomeprazole magnesium trihydrate) in the form of enteric-coated pellets. Esomeprazole sodium is also available as a sterile, freeze dried, white to off white, porous cake or powder in a 5 mL vial, intended for intravenous administration after reconstitution; NEXIUM I.V. injection contains esomeprazole sodium 21.3 mg of 42.5 mg equivalent to esomeprazole 20 mg or 40 mg.

U.S. Patent No. 6,143,771 discloses esomeprazole sodium and processes for its preparation.

U.S. Patent No. 6,875,872 discloses esomeprazole magnesium and a process for its preparation.

International Application Publication No. WO 2003/089408 discloses alkali or alkaline earth metal salts of esomeprazole, including a sodium salt.

International Application Publication No. WO 1998/54171 discloses a process for the preparation of the magnesium salt of the S-enantiomer of omeprazole trihydrate, wherein a potassium salt of S-omeprazole is used as an intermediate.

There is a general need for pharmaceutically active compounds that may be produced by processes giving products suitable for pharmaceutical preparations. Preferably, such products have one or more of the following properties: easy to handle, good dissolution rate, excellent bioavailability and good storage stability. It has been observed that many of the crystalline forms of esomeprazole or its pharmaceutically acceptable salts as well as its amorphous form can be unstable under normal storage conditions and this can result in significantly increased levels of impurities in the product over time.

Although the known esomeprazole salts address some of the deficiencies in terms of manufacturability, there remains a need for yet further improvement in these properties as well as improvements in other properties such as flowability, vapor impermeability and solubility. Further, the discovery of new crystalline polymorphic forms of a drug enlarges the repertoire of materials that a formulation scientist has with which to design a pharmaceutical dosage form of a drug with a targeted release profile or other desired characteristics.

Pharmaceutical stability depends on simultaneous influence of a variety of factors, of which some important factors are the size of crystals, shape of crystals, water assay, assay of residual solvents and assay of impurities. One or more of these factors may be uniquely characterized by the isolation process of the crystalline forms of esomeprazole salts according to the invention.

Toward this end, it has been the endeavor of pharmaceutical scientists to provide a novel and stable crystalline form of drug substances, more specifically, thermodynamically stable forms of drug substances, which would have the strengths of the crystalline forms, *viz*. thermodynamic stability, and those of the amorphous form, *viz*. enhanced solubility, rapid onset of action and an enhanced bioavailability.

There remains a continuing need to provide salts and their polymorphs of esomeprazole and processes for their preparation, which are inexpensive, readily scaleable to industrial levels.

### SUMMARY OF THE INVENTION

Aspects of the present invention relate to the esomeprazole salts and processes for preparation thereof. Embodiments of the invention include esomeprazole salts such as calcium, lithium, potassium, barium, zinc, and copper, as well as amorphous and crystalline polymorphic forms of these salts..

In another aspect, there is provided a crystalline Form C1, C2 and amorphous form of esomeprazole copper salt.

In another aspect, there is provided a crystalline Form Z1, and amorphous form of esomeprazole zinc salt.

In another aspect, there is provided a crystalline Form B1, B2, B3, B4, B5, B6, and amorphous form of esomeprazole barium salt.

In another aspect, there is provided a crystalline Form P1, P2, P3, P4, P5, P6 and P7 of esomeprazole potassium salt.

In another aspect, there is provided a crystalline Form K1, K2, K3 and K4 of esomeprazole calcium salt.

In another aspect, there is provided a crystalline Form L1, L2, L3, L4, L5, L6, L7, and amorphous form of esomeprazole lithium salt.

In one embodiment, the esomeprazole salts of the invention are provided in a pure form. In another embodiment, they are provided in a substantially pure form. In still another embodiment, they are provided in an essentially pure form.

In another aspect, there is provided a process for the preparation of esomeprazole salts or polymorphic forms thereof, the process including:
a) providing a solution comprising esomeprazole in a suitable organic solvent under suitable conditions;
b) adding a suitable base or a salt to the solution of step a) under suitable conditions;
c) recovering the solid from step b) under suitable conditions to afford the desired salt of esomeprazole or a polymorphic form thereof.

### BRIEF DESCRIPTION OF THE DRAWINGS

Fig. 1 is an illustration of X-ray powder diffraction ("XRPD") pattern of esomeprazole copper crystalline Form C1 prepared according to Example 1.
Fig. 2 is an illustration of XRPD pattern of crystalline esomeprazole copper Form C2 prepared according to Example 2.
Fig. 3 is an illustration of XRPD pattern of an amorphous form of esomeprazole copper prepared according to Example 3.
Fig. 4 is an illustration of XRPD pattern of an amorphous form of esomeprazole zinc prepared according to Example 4 and Example 5.
Fig. 5 is an illustration of XRPD pattern of esomeprazole zinc crystalline Form Z1 prepared according to Example 6.
Fig. 6 is an illustration of XRPD pattern of an amorphous form of esomeprazole barium prepared according to Example 7.
Fig. 7 is an illustration of XRPD pattern of esomeprazole barium crystalline Form B1 prepared according to Example 8.
Fig. 8 is an illustration of XRPD pattern of esomeprazole barium crystalline Form B2 prepared according to Example 9.
Fig. 9 is an illustration of XRPD pattern of esomeprazole barium crystalline Form B3 prepared according to Example 10.
Fig. 10 is an illustration of XRPD pattern of esomeprazole barium crystalline Form B4 prepared according to Example 11.
Fig. 11 is an illustration of XRPD pattern of esomeprazole barium crystalline Form B5 prepared according to Example 12.
Fig. 12 is an illustration of XRPD pattern of esomeprazole barium crystalline Form B6 prepared according to Example 13.
Fig. 13 is an illustration of XRPD pattern of esomeprazole potassium crystalline Form P1 prepared according to Example 14.
Fig. 14 is an illustration of XRPD pattern of esomeprazole potassium crystalline Form P2 prepared according to Example 15.
Fig. 15 is an illustration of XRPD pattern of esomeprazole potassium crystalline Form P3 prepared according to Example 16.
Fig. 16 is an illustration of XRPD pattern of esomeprazole potassium crystalline Form P4 prepared according to Example 17.
Fig. 17 is an illustration of XRPD pattern of esomeprazole potassium crystalline Form P5 prepared according to Example 18.
Fig. 18 is an illustration of XRPD pattern of esomeprazole potassium crystalline Form P6 prepared according to Example 19.
Fig. 19 is an illustration of XRPD pattern of esomeprazole potassium crystalline Form P7 prepared according to Example 20.
Fig. 20 is an illustration of XRPD pattern of esomeprazole calcium crystalline Form K1 prepared according to Example 21.
Fig. 21 is an illustration of XRPD pattern of esomeprazole calcium crystalline Form K2 prepared according to Example 22.
Fig. 22 is an illustration of XRPD pattern of esomeprazole calcium crystalline Form K3 prepared according to Example 23.
Fig. 23 is an illustration of XRPD pattern of esomeprazole calcium crystalline Form K4 prepared according to Example 24.
Fig. 24 is an illustration of XRPD pattern of an amorphous form of esomeprazole lithium prepared according to Example 25.
Fig. 25 is an illustration of XRPD pattern of esomeprazole lithium crystalline Form L1 prepared according to Example 26.
Fig. 26 is an illustration of XRPD pattern of esomeprazole lithium crystalline Form L2 prepared according to Example 27.
Fig. 27 is an illustration of XRPD pattern of esomeprazole lithium crystalline Form L3 prepared according to Example 28.
Fig. 28 is an illustration of XRPD pattern of esomeprazole lithium crystalline Form L4 prepared according to Example 29.
Fig. 29 is an illustration of XRPD pattern of esomeprazole lithium crystalline Form L5 prepared according to Example 30.
Fig. 30 is an illustration of XRPD pattern of esomeprazole lithium crystalline Form L6 prepared according to Example 31.
Fig. 31 is an illustration of XRPD pattern of esomeprazole lithium crystalline Form L7 prepared according to Example 32.

### DETAILED DESCRIPTION OF THE INVENTION

While the specification concludes with the claims particularly pointing and distinctly claiming the invention, it is believed that the present invention will be better understood from the following description. All percentages and ratios used herein are by weight of the total composition and all measurements made are at 25°C and normal pressure unless otherwise designated. All temperatures are in Degrees Celsius unless specified otherwise. The present invention can comprise (open ended) or consist essentially of the components of the present invention as well as other ingredients or elements described herein. As used herein, "comprising" means the elements recited, or their equivalent in structure or function, plus any other element or elements which are not recited. The terms "having" and "including" are also to be construed as open ended unless the context suggests otherwise. As used herein, "consisting essentially of means that the invention may include ingredients in addition to those recited in the claim, but only if the additional ingredients do not materially alter the basic and novel characteristics of the claimed invention. Preferably, such additives will not be present at all or only in trace amounts. However, it may be possible to include up to about 10% by weight of materials that could materially alter the basic and novel characteristics of the invention as long as the utility of the compounds (as opposed to the degree of utility) is maintained. All ranges recited herein include the endpoints, including those that recite a range "between" two values. Terms such as "about," "generally," "substantially," and the like are to be construed as modifying a term or value such that it is not an absolute, but does not read on the prior art. Such terms will be defined by the circumstances and the terms that they modify as those terms are understood by those of skill in the art. This includes, at very least, the degree of expected experimental error, technique error and instrument error for a given technique used to measure a value.

Note that while the specification and claims may refer to a final product such as, for example, a tablet or other dosage form of the invention as, for example, containing particles having a certain particle size or distribution, or a certain type of, for example, a specific form of a filler, it may be difficult to tell from the final dosage form that the recitation is satisfied. However, such a recitation may be satisfied if the materials used prior to final production (in the case of a tablet for example, blending and tablet formulation), for example, meet that recitation. Indeed, as to any property or characteristic of a final product which cannot be ascertained from the dosage form directly, it is sufficient if that property resides in the components recited just prior to final production steps.

Where this document refers to a material, such as in this instance, esomeprazole, and the unique crystalline forms, salts, solvates and/or optical isomers thereof by reference to patterns, spectra or other graphical data, it may do so by qualifying that they are "substantially" shown or depicted in a Figure, or by one or more data points. By "substantially" used in such a context, it will be appreciated that patterns, spectra and other graphical data can be shifted in their positions, relative intensities, or other values due to a number of factors known to those of skill in the art. For example, in the crystallographic and powder X-ray diffraction arts, shifts in peak positions or the relative intensities of one or more peaks of a pattern can occur because of, without limitation: the equipment used, the sample preparation protocol, preferred packing and orientations, the radiation source, operator error, method and length of data collection, and the like. However, those of ordinary skill in the art should be able to compare the figures herein with a pattern generated of an unknown form of, in this case, esomeprazole, and confirm its identity as one of the forms disclosed and claimed herein. The same holds true for other techniques which may be reported herein.

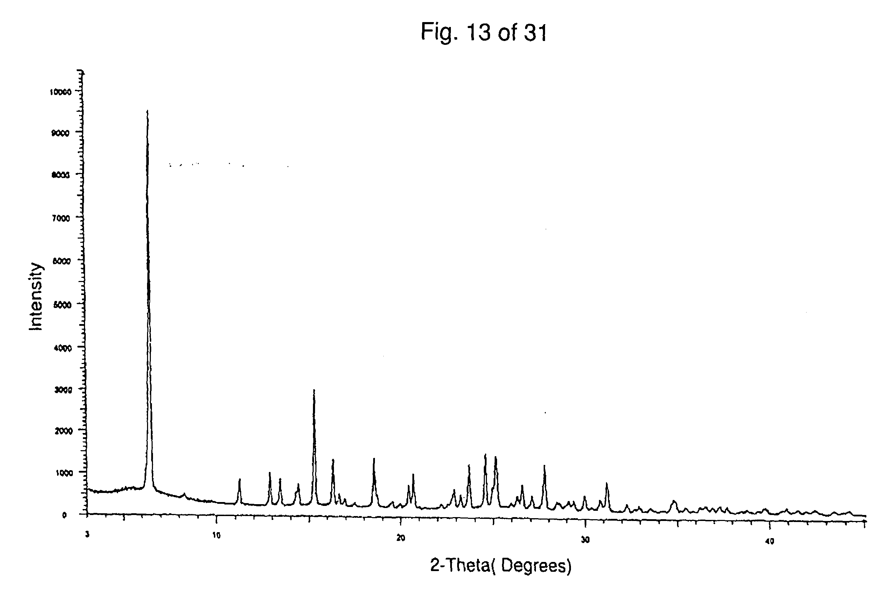

In addition, where a reference is made to a figure, it is permissible to, and this document includes and contemplates, the selection of any number of data points illustrated in the figure which uniquely define that crystalline form, salt, solvate, and/or optical isomer, within any associated and recited margin of error, for purposes of identification. Again, and for example, for a crystalline form of esomeprazole, it is permissible to select any number of PXRD peaks represented in Figure 13, often between 4 and 10, which +/- 0.2 degrees two theta, uniquely identify that form, as a way of describing and claiming that material.

A reference to a molecule such as, in this case, esomeprazole salt, unless otherwise specified or inconsistent with the disclosure in general, refers to any crystalline or amorphous form, optical isomer and/or solvate form thereof.

When a molecule or other material is identified herein as "pure", it generally means, unless specified otherwise, that the material is about 99% pure or more. In general, this refers to purity with regard to unwanted residual solvents, reaction byproducts, impurities and unreacted starting materials. In the case of stereoisomers, "pure" also means 98% of one enantiomer or diastereomer, as appropriate. "Substantially" pure means, the same as "pure" except that the lower limit is about 95% pure or more and likewise, "essentially" pure means the same as "pure" except that the lower limit is about 90% pure.

In one aspect, the present invention provides a process for the preparation of esomeprazole salts or polymorphic forms thereof, the process including:
a) providing a solution comprising esomeprazole in a suitable organic solvent under suitable conditions;
b) adding a suitable base or a salt to the solution of step a) under suitable conditions;
c) recovering the solid from step b) under suitable conditions to afford the desired salt of esomeprazole or a polymorphic form thereof.

Step a) involves providing a solution comprising esomeprazole, often in free form, in a suitable organic solvent under suitable conditions.

The solution of esomeprazole may be obtained by dissolving esomeprazole free base in a suitable solvent, a solution may be obtained directly from a reaction in which esomeprazole free base is formed, or by any other method.

Suitable organic solvents that may be used to dissolve the free base include but are not limited to: C₁-C₄ alcohols such as methanol, ethanol, propanol, n-butanol, isopropyl alcohol and the like; C₂-C₆ ketone solvents such as acetone, ethyl methyl ketone, and diethyl ketone; chlorinated solvents, such as C₁-C₆ straight chain or branched chlorohydrocarbons including ethylene dichloride, dichloromethane, chloroform, carbon tetrachloride, chlorobenzene, dichlorobenzene and the like; ethers such as tetrahydrofuran ("THF"), diethyl ether, methyl tertiary-butyl ether, 1,4-dioxane and the like; hydrocarbon solvents such as toluene, xylene, cyclohexane and the like; esters such as ethyl acetate, isopropyl acetate, tertiary-butyl acetate and the like; nitriles such as acetonitrile, propionitrile and the like; aprotic polar solvents such as dimethyl sulfoxide ("DMSO"), N,N-dimethylformamide ("DMF"), dimethyl acetamide ("DMAC"), N-methylpyrrolidone ("NMP") and the like; and mixtures thereof or their combination with water in various proportions. Any solvent is acceptable as long as it provides a clear solution of the esomeprazole, at the temperature of operation.

The dissolution temperatures may range from about 20 to about 100° C depending on the solvent used for dissolution. Any other temperature is also acceptable as long as the stability of esomeprazole free base is not compromised and a clear solution is obtained.

The maintenance time for dissolution to occur for getting a homogenous solution may be any desired time periods to achieve the desired product yield and purity, times from about 1 to 10 hours, or longer, frequently being adequate.

Step b) involves adding a suitable base or a salt to the solution of step a) under suitable conditions.

Suitable metal hydroxides or their salts that may be used as bases for salt formation include but are not limited to lithium hydroxide, copper sulfate, barium hydroxide, zinc chloride, potassium hydroxide, calcium hydroxide, and the like.

A solution of the base(s) may be prepared by dissolving them in suitable solvents such as, but not limited to: water; and C₁-C₄ alcohols such as methanol, ethanol, propanol, n-butanol, isopropyl alcohol and the like.

Suitable temperatures for addition of metal salt solution range from about 10°C to about 50°C or from about 20°C to about 30°C.

The mixtures of esomeprazole and the base may be maintained for any desired time periods to achieve the desired product yield and purity, times from about 1 to 10 hours, or longer, frequently being adequate.

Stirring or other alternate methods such as shaking, agitation and the like, that mix the contents is done for crystallization to occur, the reaction mass may be maintained further at temperatures lower than the concentration temperatures such as for example below about 10°C to about 25 °C, for a period of time as required for a more complete formation of the product. The exact cooling temperature and time required for complete crystallization may be readily determined by a person skilled in the art and will also depend on parameters such as concentration and temperature of the solution or slurry.

Optionally crystallization may be initiated by methods such as cooling, seeding, partial removal of the solvent from the solution, by adding an anti-solvent to the solution or a combination thereof.

Step c) involves recovering the solid from step b) under suitable conditions to afford the desired salt of esomeprazole or polymorphic forms thereof.

The method by which the solid material is recovered from the final mixture, with or without cooling below the operating temperature, may be any of techniques such as filtration by gravity or by suction, centrifugation, or slow evaporation and the like. The solids (crystalline and/or amorphous) so isolated will carry a small proportion of occluded mother liquor containing a higher percentage of impurities. If desired the crystals may be washed with a solvent to wash out the mother liquor or may be recrystallized.

The wet cake obtained in step c) may optionally be further dried. Drying may be suitably carried out in a tray dryer, vacuum oven, air oven, fluidized bed drier, spin flash dryer, flash dryer and the like. The drying may be carried out at temperatures of about 35°C to about 90°C with or without vacuum. Drying may be carried out for any desired time until the required product purity is achieved, time periods from about 1 to 20 hours, or longer, frequently being sufficient.

In another aspect, the present invention provides different polymorphic forms of various salts of esomeprazole characterized by PXRD pattern and their illustrative figures as depicted in Table 1.

**Table 1**

| **Polymorphic Form** | **Characteristic PXRD peaks** **(± 0.2 degrees 2 theta)** | **Illustrative Figure** |
|---|---|---|
| Crystalline Form C1 of Esomeprazole copper salt | 5.5, 22.6, 23.5, 25.5, 31.8, 33.9, and 37.8 | Fig. 1 |
| Crystalline Form C2 of Esomeprazole copper salt | 18.6, 19.0, 22.6, and 37.8 | Fig. 2 |
| Amorphous Esomeprazole copper salt | --- | Fig. 3 |
| Amorphous esomeprazole zinc salt | --- | Fig. 4 |
| Crystalline Form Z1 of esomeprazole zinc salt | 27.2 and 31.6 | Fig. 5 |
| Amorphous form of esomeprazole barium salt | --- | Fig. 6 |
| Crystalline Form B1 of esomeprazole barium salt | 16.0, 16.2, and 24.3 | Fig. 7 |
| Crystalline Form B2 of esomeprazole barium salt | 5.5, 16.0, 16.2, 21.1, 27.3, 27.3, 39.2 | Fig. 8 |
| Crystalline Form B3 of esomeprazole barium salt | 5.4, 6.4, 15.9, 16.2, 17.8, 24.3, 24.3, 26.0, and 28.1 | Fig. 9 |
| Crystalline Form B4 of esomeprazole barium salt | 5.7, 13.4, 16.0, 16.3, 21.2, 24.4, 23.8, and 27.3 | Fig. 10 |
| Crystalline Form B5 of esomeprazole barium salt | 5.5, 13.3, 15.9, 16.2, 24.7, 26.0, and 27.2 | Fig. 11. |
| Crystalline Form B6 of esomeprazole barium salt | 5.5, 6.9, 13.4, 15.9, 16.2, 21.2, 24.8, and 27.2 | Fig. 12 |
| Crystalline Form P1 of esomeprazole potassium salt | 6.3, 16.2, 18.5, 23.7, 24.6, 25.1, and 27.8 | Fig. 13 |
| Crystalline Form P2 of esomeprazole potassium salt | 7.0, 17.5, 18.8, 22.5, 22.8, 28.7, 29.2, 33.6, 35.4, 35.6, and 36.2 | Fig. 14 |
| Crystalline Form P3 of esomeprazole potassium salt | 7.5, 14.3, 18.3, 18.9, 19.9, 21.3, 22.4, 22.9, 25.8, and 31.1 | Fig. 15 |
| Crystalline Form P4 of esomeprazole potassium salt | 7.5, 14.3, 18.3, 18.9, 19.9, 21.3, 22.4, 22.9, 25.8, and 31.13 | Fig. 16 |
| Crystalline Form P5 of esomeprazole potassium salt | 7.5, 14.9, 18.9,19.9, and 22.9 | Fig. 17 |
| Crystalline Form P6 of esomeprazole potassium salt | 7.4, 18.2, 21.3, 23.0, and 31.02 | Fig. 18 |
| Crystalline Form P7 of esomeprazole potassium salt | 5.2, 7.2, 18.1, 18.9, and 22.8 | Fig. 19 |
| Crystalline Form K1 of esomeprazole calcium salt | 5.4, 11.4, 13.0, and 24.2 | Fig. 20 |
| Crystalline Form K2 of esomeprazole calcium salt | 6.4, 6.7, 26.1, and 31.6 | Fig. 21 |
| Crystalline Form K3 of esomeprazole calcium salt | 5.9, 6.2, 8.8, 9.3, 14.6, 26.1, 27.3, and 31.6 | Fig. 22 |
| Crystalline Form K4 of esomeprazole calcium salt | 4.0, 5.8, 6.8, 9.4, 14.7, 27.4, and 31.7 | Fig. 23 |
| Amorphous form of esomeprazole lithium salt | --- | Fig. 24 |
| Crystalline Form L1 of esomeprazole lithium salt | 6.3 | Fig. 25 |
| Crystalline Form L2 of esomeprazole lithium salt | 5.6, 6.3, 17.2, 22.7, 23.1, and 37.1 | Fig. 26 |
| Crystalline Form L3 of esomeprazole lithium salt | 4.9, 6.4, and 22.9 | Fig. 27 |
| Crystalline Form L4 of esomeprazole lithium salt | 5.4, 6.3, 18.3, 19.0, 22.5, 24.1, 27.8, and 22.8 | Fig. 28 |
| Crystalline Form L5 of esomeprazole lithium salt | 5.5, 6.3, 22.6, and 22.9 | Fig. 29 |
| Crystalline Form L6 of esomeprazole lithium salt | 5.5, 6.2, 11.1, 17.1, 18.1, 18.9, 20.3, 22.5, 23.0, 27.9, 32.2, and 26.9 | Fig. 30 |
| Crystalline Form L7 of esomeprazole lithium salt | 5.4, 6.2, 10.9, 12.2, 16.9, 17.2, 18.2, 19.2, 22.3, 22.7, and 28.8 | Fig. 31 |

X-ray powder diffraction (PXRD) patterns reported herein were determined using a Bruker AXS D8 Advance powder X-ray diffractometer with a Cu K alpha radiation source. X-ray powder diffraction patterns were obtained using a Bruker X-Ray powder diffractometer, goniometer model 1050/70 at a scanning speed of 1 degree per minute, with a Cu radiation of λ=1.5418 Å.

The starting material for the present processes may be esomeprazole obtained by any method. The starting material for a process may also be in any polymorphic form, such as amorphous or crystalline forms of esomeprazole or a mixture of amorphous and crystalline forms of esomeprazole obtained by any method.

Optionally, seed crystals of polymorphic crystalline forms of esomeprazole salts may also be used in a process, to initiate crystallization.

In yet another embodiment of the present invention there is provided pharmaceutical compositions containing a therapeutically effective amount of esomeprazole salts of the present invention including, but not limited to, the amorphous and crystalline salts described herein, together with one or more pharmaceutically acceptable excipients.

Dosage forms such as tablets, capsules and gel caps may include between about 0.01 micrograms and about 2 grams of the esomeprazole salts of the invention. However, each dosage form typically includes between 1 to about 100 mg, more preferably about 10 to about 50 mg. The daily dose of esomeprazole will vary with the size, age and condition of the patient, the dosage forms available and the sound medical judgment of the health professionals involved. Doses may be once a day or divided into 2 or more daily doses. The dose may be 10 to 40 mg given once a day, up to 80 mg, divided into two daily doses and the like.

The pharmaceutical composition comprising esomeprazole salts of the invention along with one or more pharmaceutically acceptable excipients may be formulated as: solid oral dosage forms such as, but not limited to, powders, granules, pellets, tablets, and capsules; liquid oral dosage forms such as but not limited to syrups, suspensions, dispersions, and emulsions; and injectable preparations such as but not limited to solutions, dispersions, and freeze dried compositions. Formulations may be in the form of immediate release, delayed release or modified release. Further, immediate release compositions may be conventional, dispersible, chewable, mouth dissolving, or flash melt preparations, and modified release compositions that may comprise hydrophilic or hydrophobic, or combinations of hydrophilic and hydrophobic, release rate controlling substances to form matrix or reservoir systems or combinations of matrix and reservoir systems. The compositions may be prepared by direct blending, dry granulation, wet granulation or by extrusion and spherinization. Compositions may be presented as uncoated, film coated, sugar coated, powder coated, enteric coated or modified release coated. Compositions of the present invention may further comprise one or more pharmaceutically acceptable excipients.

Pharmaceutically acceptable excipients that find use in the present invention include, but are not limited to: diluents such as starch, pregelatinized starch, lactose, powdered cellulose, microcrystalline cellulose, dicalcium phosphate, tricalcium phosphate, mannitol, sorbitol, sugar and the like; binders such as acacia, guar gum, tragacanth, gelatin, polyvinylpyrrolidone, hydroxypropyl cellulose, hydroxypropyl methylcellulose, pregelatinized starch and the like; disintegrants such as starch, sodium starch glycolate, pregelatinized starch, crospovidone, croscarmellose sodium, colloidal silicon dioxide and the like; lubricants such as stearic acid, magnesium stearate, zinc stearate and the like; glidants such as colloidal silicon dioxide and the like; solubility or wetting enhancers such as anionic or cationic or neutral surfactants; complex forming agents such as various grades of cyclodextrins, resins; release rate controlling agents such as hydroxypropyl cellulose, hydroxymethyl cellulose, hydroxypropyl methylcellulose, ethyl cellulose, methylcellulose, various grades of waxes, methyl methacrylates, and the like. Other pharmaceutically acceptable excipients that are of use include but are not limited to film formers, plasticizers, colorants, flavoring agents, sweeteners, viscosity enhancers, preservatives, antioxidants and the like.

The processes of present invention are simple, cost-effective, eco-friendly, reproducible, scalable, and robust to produce esomeprazole salts with high purity.

Certain specific aspects and embodiments of the present invention will be explained in greater detail with reference to the following examples, which are provided by way of illustration only and should not be construed as limiting the scope of the invention in any manner.

### EXAMPLES

### EXAMPLE 1: PREPARATION OF ESOMEPRAZOLE COPPER CRYSTALLINE FORM C1

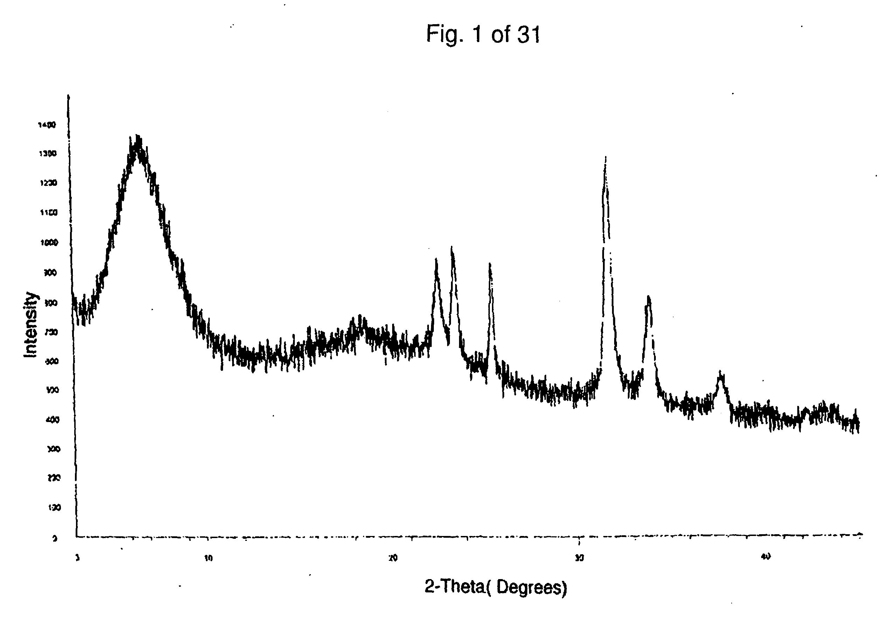

Esomeprazole free base (20 gm) of Formula I and n-butanol (200 ml) were charged into a clean and dry 4 neck round bottom flask followed by stirring for 10 minutes. Copper sulfate (12.2 gm) dissolved in methanol (100 ml) was added slowly at 25-30°C over a period of 10-20 minutes and stirred for 60 minutes. The formed solid was filtered and the solid was washed with n-butanol (100 ml). Solid obtained was dried at 50°C under vacuum for 2-3 hours to afford 16 g of the title compound, characterized by a PXRD pattern substantially in accordance with Fig. 1.

### EXAMPLE 2: PREPARATION OF ESOMEPRAZOLE COPPER CRYSTALLINE FORM C2

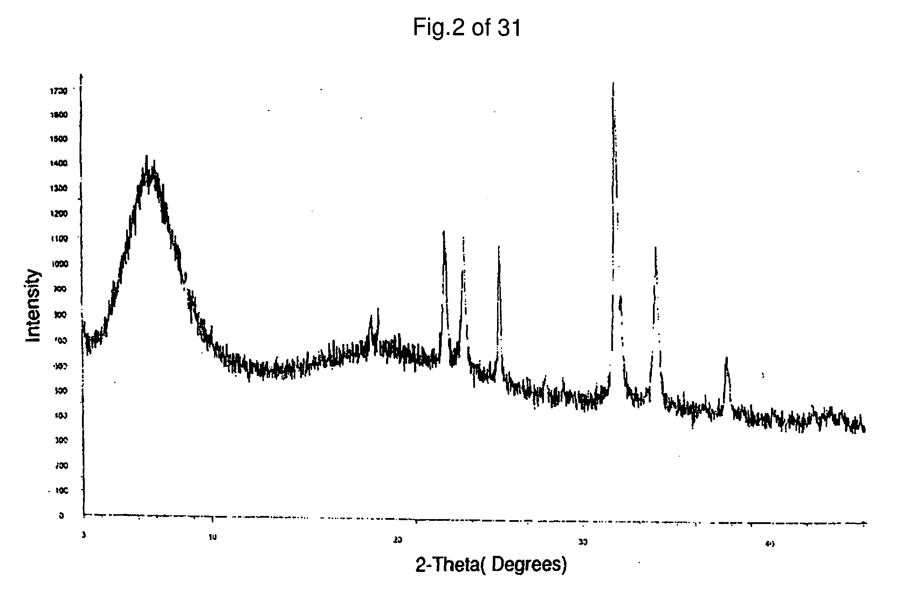

Esomeprazole free base (20 gm) of Formula I and N,N-dimethylformamide (DMF) (200 ml) were charged into a clean and dry 4 neck round bottom flask followed by stirring for 10 minutes. Copper sulfate (12.2 g) dissolved in methanol (100 ml) was added slowly at 25-30°C over a period of 10-20 minutes and stirred for 60 minutes. The formed solid was filtered and the solid was washed with methanol (100 ml). Solid obtained was dried at 50°C under vacuum for 2-3 hours to afford 17 g of the title compound, characterized by a PXRD pattern substantially in accordance with Fig. 2.

### EXAMPLE 3: PREPARATION OF ESOMEPRAZOLE COPPER AMORPHOUS FORM

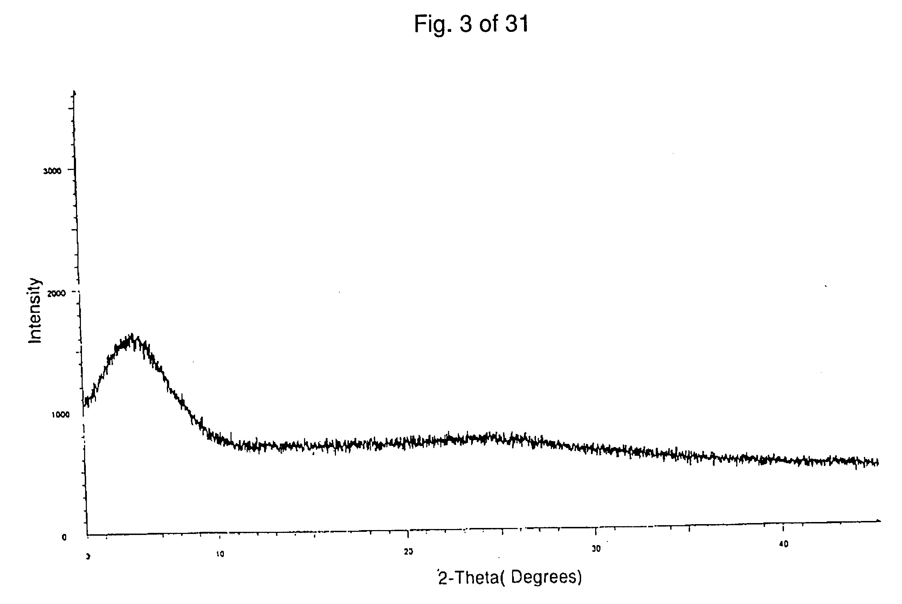

Esomeprazole free base (20 gm) of Formula I and dichloromethane (100 ml) were charged into a clean and dry 4 neck round bottom flask followed by stirring for 10 minutes. Copper sulfate (6.1 gm) dissolved in methanol (50 ml) was added slowly at 25-30°C over a period of 10-20 minutes and stirred for 3 hours. The solvents were distilled completely at 40°C under vacuum to afford 5.8 g of the title compound, characterized by a PXRD pattern substantially in accordance with Fig. 3.

### EXAMPLE 4: PREPARATION OF ESOMEPRAZOLE ZINC AMORPHOUS FORM

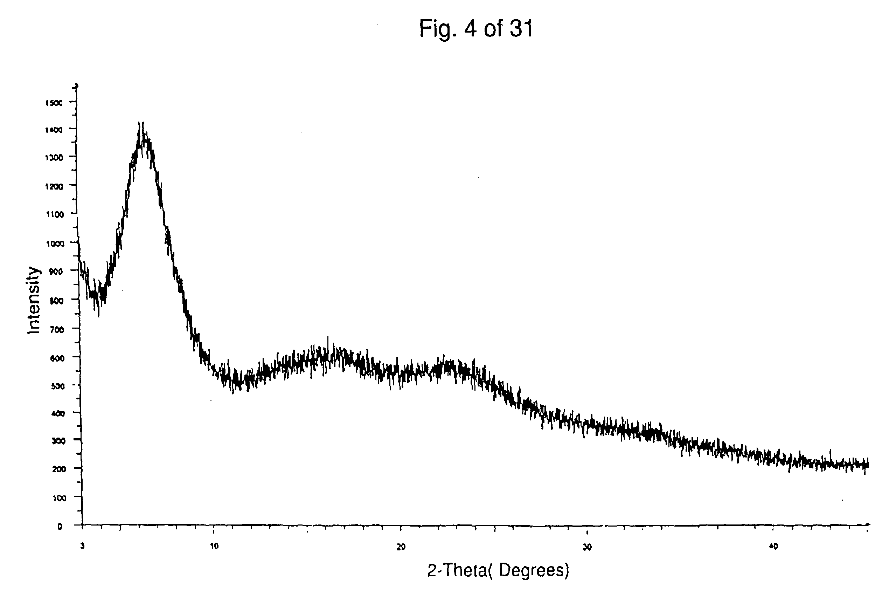

Esomeprazole free base (10 gm) of Formula I and water (100 ml) were charged into a clean and dry 4 neck round bottom flask followed by stirring for 10 minutes. Zinc sulfate (5.2 gm) dissolved in water (100 ml) was added slowly at 25-30°C over a period of 10-20 minutes and stirred for 3 hours. The formed solid was filtered and the solid was washed with water (50 ml). Solid obtained was dried at 50°C under vacuum for 2-3 hours to afford 16 g of the title compound, characterized by an X-ray powder diffraction pattern substantially in accordance with Fig. 4.

### EXAMPLE 5: PREPARATION OF ESOMEPRAZOLE ZINC AMORPHOUS FORM

Esomeprazole free base (20 gm) of Formula I and water (200 ml) were charged into a clean and dry 4 neck round bottom flask followed by stirring for 10 minutes. zinc chloride (5.2 gm) dissolved in water (100 ml) was added slowly at 25-30°C over a period of 10-20 minutes and stirred for 3 hours. The formed solid was filtered and the solid was washed with water (100 ml). The solid obtained was dried at 25-30°C for 3-4 hours to afford 37 g of the title compound, characterized by a PXRD pattern substantially in accordance with Fig. 4.

### EXAMPLE 6: PREPARATION OF ESOMEPRAZOLE ZINC CRYSTALLINE FORM Z1

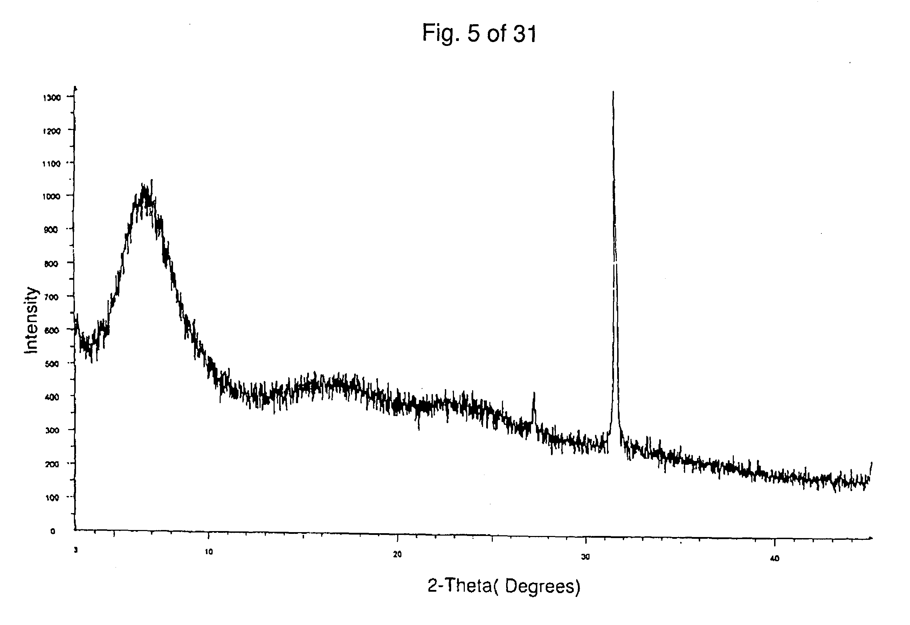

Esomeprazole free base (20 gm) of Formula I and methanol (200 ml) were charged into a clean and dry 4 neck round bottom flask followed by stirring for 10 minutes. Zinc chloride (5.2 gm) dissolved in water (100 ml) was added slowly at 25-30°C over a period of 10-20 minutes and stirred for 2-3 hours. The formed solid was filtered and washed with water (50 ml). The solid was dried under vacuum at 50°C to afford 16 g of the title compound, characterized by a PXRD pattern substantially in accordance with Fig. 5.

### EXAMPLE 7: PREPARATION OF ESOMEPRAZOLE BARIUM AMORPHOUS FORM

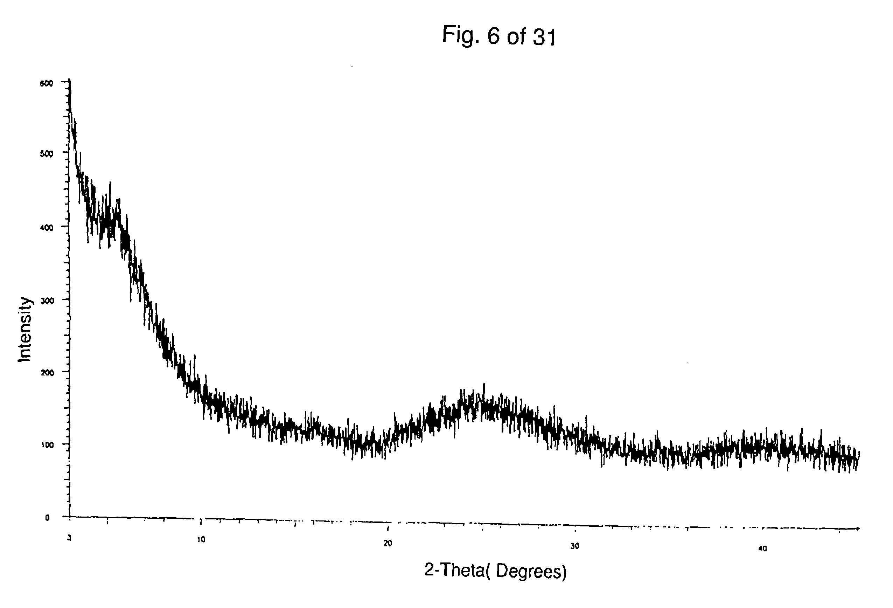

Esomeprazole free base (10 gm) of Formula I and ethyl acetate (100 ml) were charged into a clean and dry 4 neck round bottom flask followed by stirring for 10 minutes. Barium hydroxide (9.1 gm) dissolved in methanol (20 ml) was added slowly at 25-30°C over a period of 10-20 minutes and stirred for 3 hours. The formed solid was filtered and the solid was washed with ethyl acetate (40 ml). The solid obtained was dried at 50-60°C under vacuum to afford 9 g of the title compound, characterized by a PXRD pattern substantially in accordance with Fig. 6.

### EXAMPLE 8: PREPARATION OF ESOMEPRAZOLE BARIUM CRYSTALLINE FORM B1

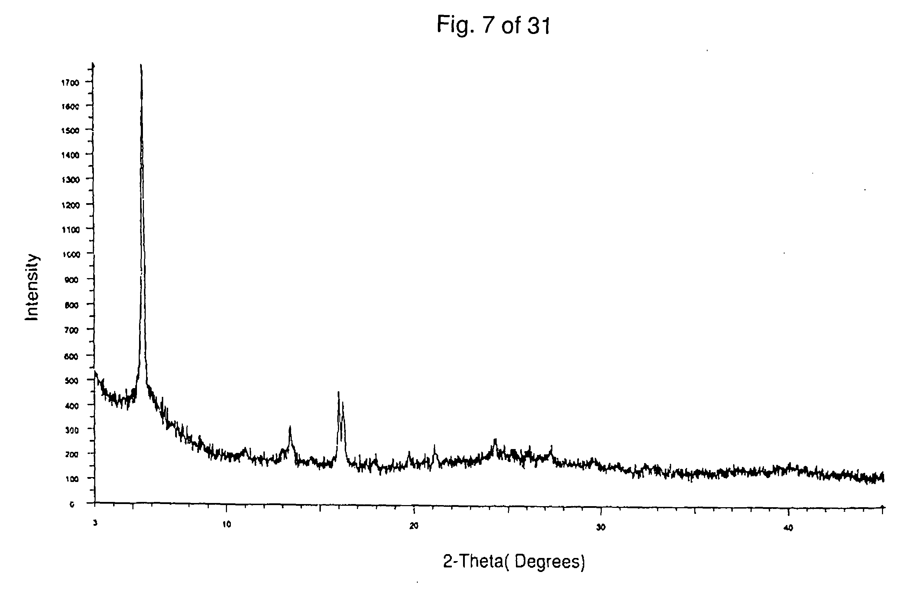

Barium hydroxide (9.1 g) and methanol (100 ml) were charged into a clean and dry 4 neck round bottom flask followed by stirring for a period of 10 minutes. Esomeprazole free base (10 g) of Formula I dissolved in methanol (100 ml) was added slowly at 25-30°C over a period of 10-20 minutes and stirred for 3 hours. The obtained solution was poured into a glass tray and allowed to evaporate to dryness overnight to afford 14 g of the title compound, characterized by a PXRD pattern substantially in accordance with Fig. 7.

### EXAMPLE 9: PREPARATION OF ESOMEPRAZOLE BARIUM CRYSTALLINE FORM B2

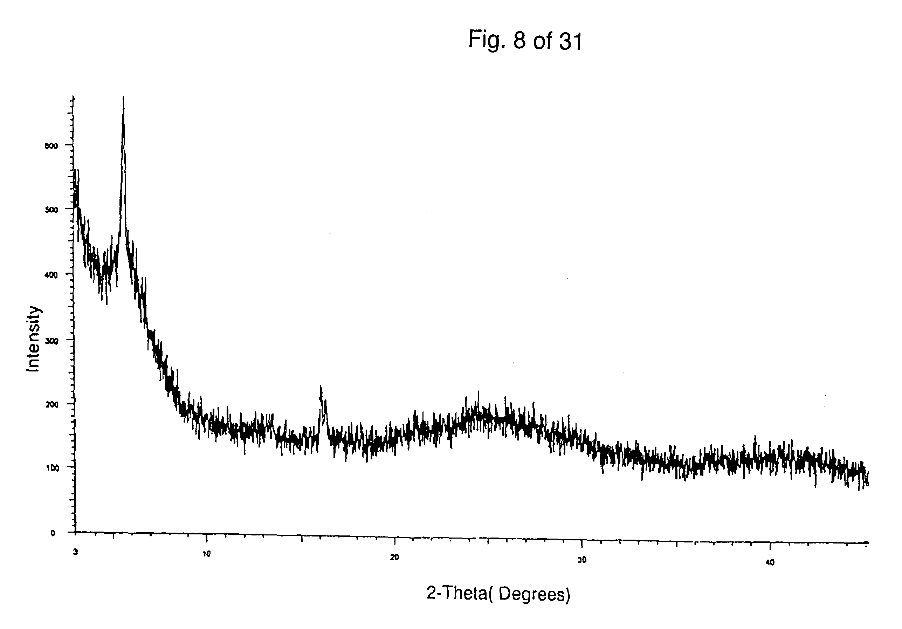

Barium hydroxide (9.1 g) and acetone (40 ml) was charged into a clean and dry 4 neck round bottom flask followed by stirring for 10 minutes. Esomeprazole free base (10 g) of Formula I dissolved in acetone (100 ml) was added slowly at 25-30°C over a period of 10-20 minutes and stirred for 3 hours. The obtained solution was poured into a glass tray and allowed to evaporate to dryness overnight to afford 13 g of the title compound, characterized by a PXRD pattern substantially in accordance with Fig. 8.

### EXAMPLE 10: PREPARATION OF ESOMEPRAZOLE BARIUM CRYSTALLINE FORM B3

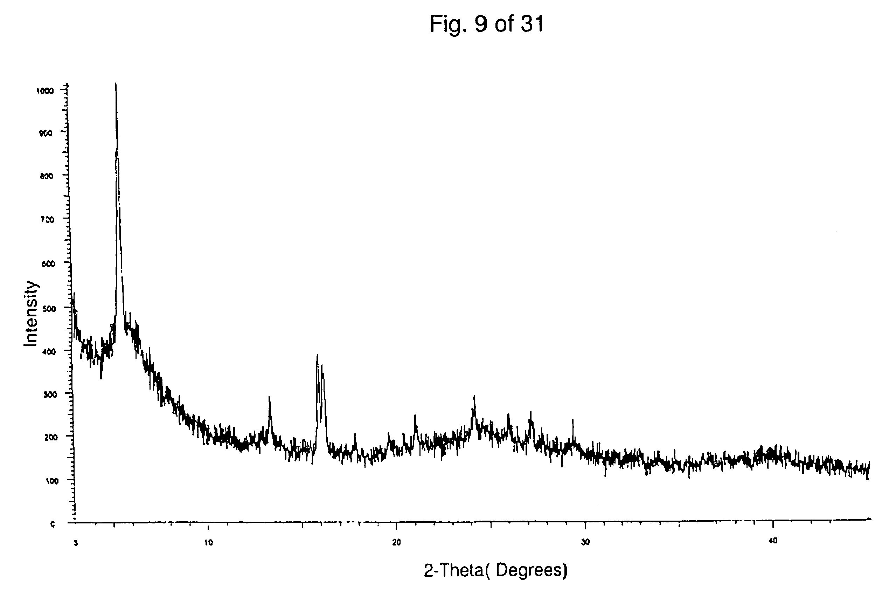

Barium hydroxide (9.1 g) and methanol (20 ml) was charged into a clean and dry 4 neck round bottom flask and stirred for 10 minutes. Esomeprazole free base of Formula I (10 g) dissolved in toluene (100 ml) was added slowly at 25-30°C over a period of 10-20 minutes and stirred for 3 hours. The obtained solution was poured into a glass tray and allowed to evaporate to dryness overnight to afford 13.3 g of the title compound, characterized by a PXRD pattern substantially in accordance with Fig. 9.

### EXAMPLE 11: PREPARATION OF ESOMEPRAZOLE BARIUM CRYSTALLINE FORM B4

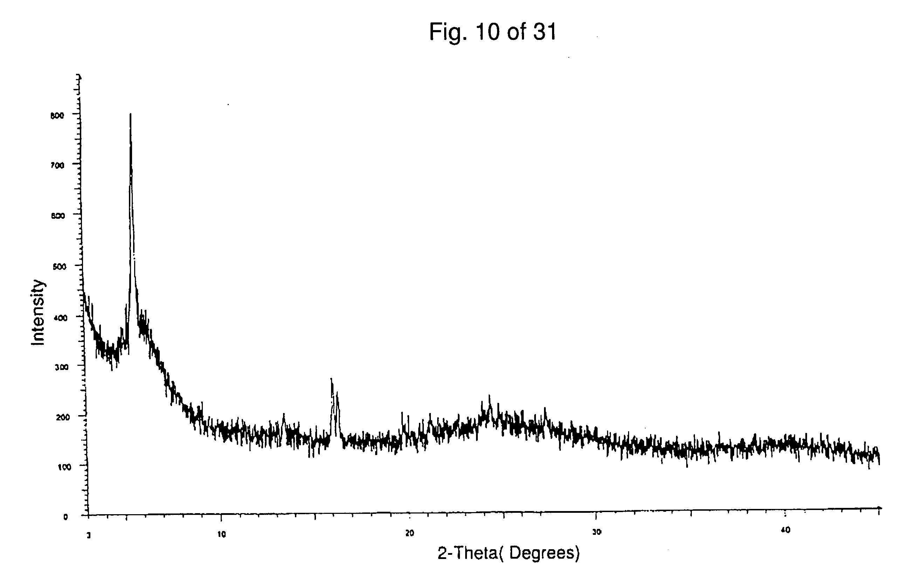

Barium hydroxide (9.1 g) and methanol (20 ml) was charged into a clean and dry 4 neck round bottom flask followed by stirring for 10 minutes. Esomeprazole free base (10 g) of Formula I dissolved in dichloromethane (100 ml) was added slowly at 25-30°C over a period of 10-20 minutes and stirred for 3 hours. The obtained solution was poured in to a glass tray and allowed to evaporate to dryness overnight to afford 13.4 g of the title compound, characterized by a PXRD pattern substantially in accordance with Fig. 10.

### EXAMPLE 12: PREPARATION OF ESOMEPRAZOLE BARIUM CRYSTALLINE FORM B5

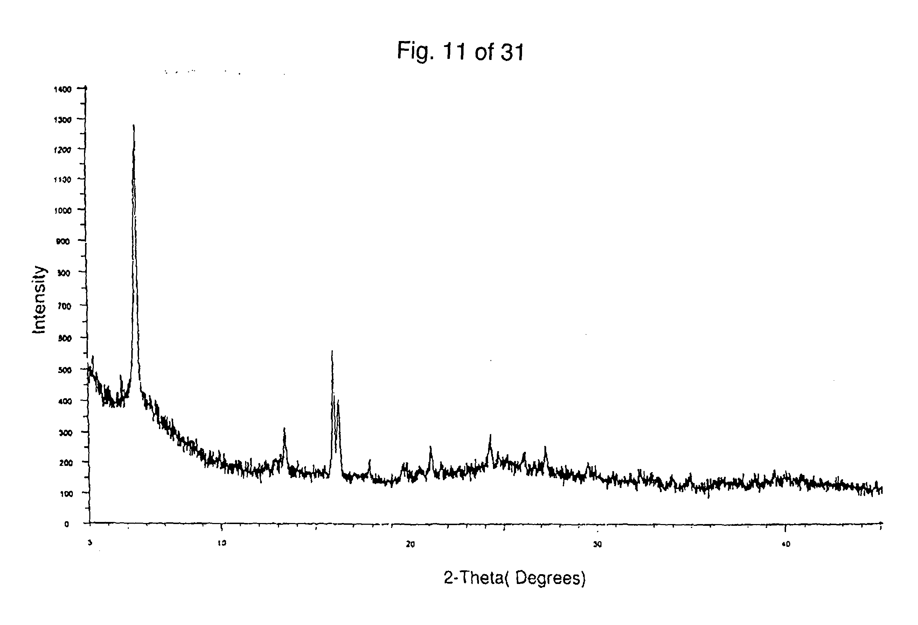

Barium hydroxide (9.1 gm) and methanol (20 ml) were charged into a clean and dry 4 neck round bottom flask followed by stirring for 10 minutes. Esomeprazole free base (10 g) of Formula I dissolved in tetrahydrofuran (THF) (100 ml) was added slowly at 25-30°C over a period of 10-20 minutes and stirred for 3 hours. The obtained solution was poured into a glass tray and allowed to evaporate to dryness overnight to afford 14 g of the title compound, characterized by a PXRD pattern substantially in accordance with Fig. 11.

### EXAMPLE 13: PREPARATION OF ESOMEPRAZOLE BARIUM CRYSTALLINE FORM B6

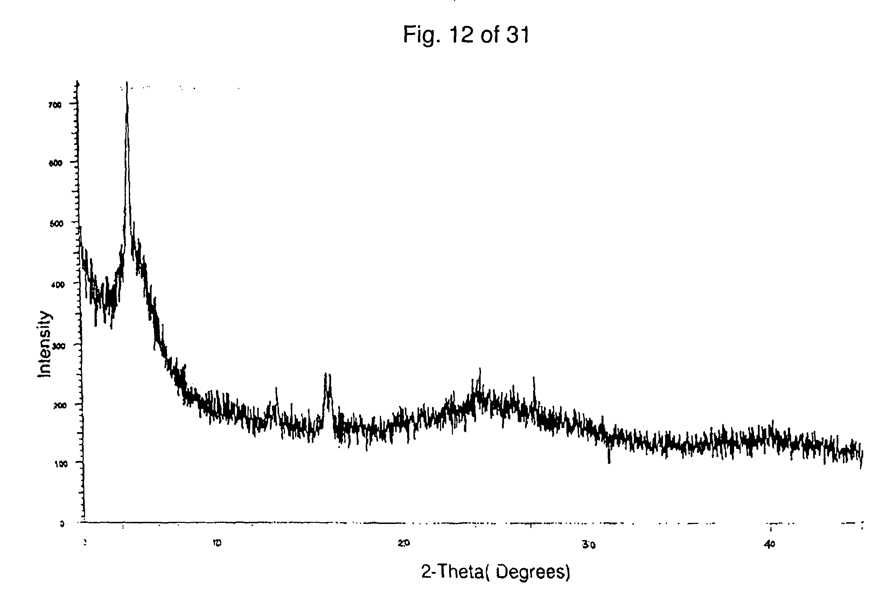

Barium hydroxide (9.1 gm) and methanol (20 ml) were charged into a clean and dry 4 neck round bottom flask, followed by stirring for 10 minutes. Esomeprazole free base (10 gm) of Formula I dissolved in acetonitrile (100 ml) was added slowly at 25-30°C over a period of 10-20 minutes and stirred for 2-3 hours. The solution obtained was poured into a glass tray and allowed to evaporate to dryness overnight to afford 12.2 g of the title compound, characterized by a PXRD pattern substantially in accordance with Fig. 12.

### EXAMPLE 14: PREPARATION OF ESOMEPRAZOLE POTASSIUM CRYSTALLINE FORM P1

Potassium hydroxide (16.2 gm) and methanol (300 ml) were charged into a clean and dry 4 neck round bottom flask followed by stirring for 10-15 minutes. Esomeprazole free base (100 g) of Formula I dissolved in methanol (150 ml) was added slowly over a period of 10-20 minutes. Toluene (150 ml) was added slowly for 10 minutes and stirred for 12 hours. Formed solid was filtered and the solid was washed with toluene (100 ml). Solid obtained was dried at 40-50°C under vacuum to afford 80 g of the title compound, characterized by a PXRD pattern substantially in accordance with Fig. 13.

### EXAMPLE 15: PREPARATION OF ESOMEPRAZOLE POTASSIUM CRYSTALLINE FORM P2

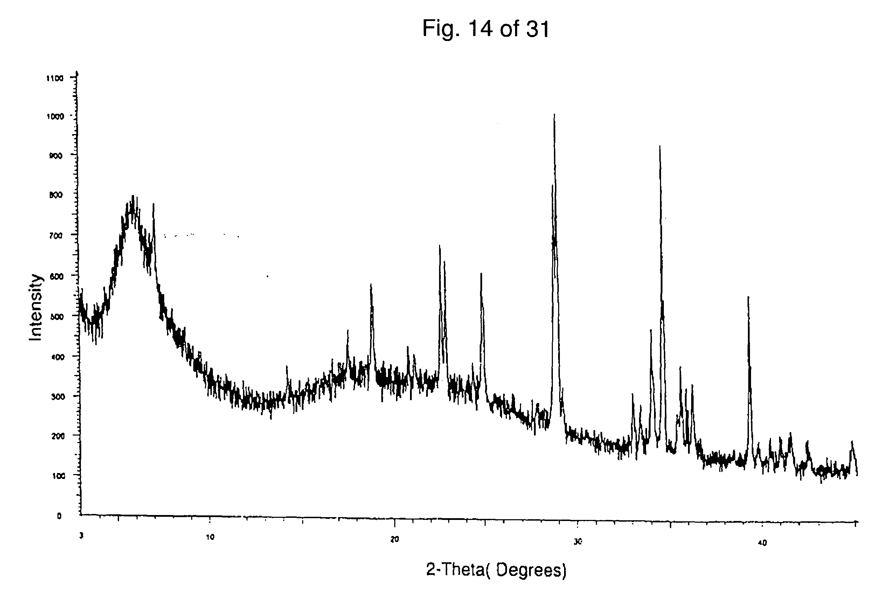

Esomeprazole free base (13.3 gm) of Formula I and ethyl acetate (85.2 ml) was charged into a clean and dry 4 neck round bottom flask followed by stirring for 10 minutes. Potassium hydroxide (1.72 gm) dissolved in 4 ml of water was added slowly at 25-30°C over a period of 10-20 minutes and stirred for 2-3 hours followed by addition of methanol (2 ml). The resulting solution was stirred at 20-30°C for 2-3 hours. The solution was distilled completely at 50°C under vacuum to afford a foamy yellow solid that was scraped from the flask to afford 11.2 g of the title compound, characterized by a PXRD pattern substantially in accordance with Fig. 14.

### EXAMPLE 16: PREPARATION OF ESOMEPRAZOLE POTASSIUM CRYSTALLINE FORM P3

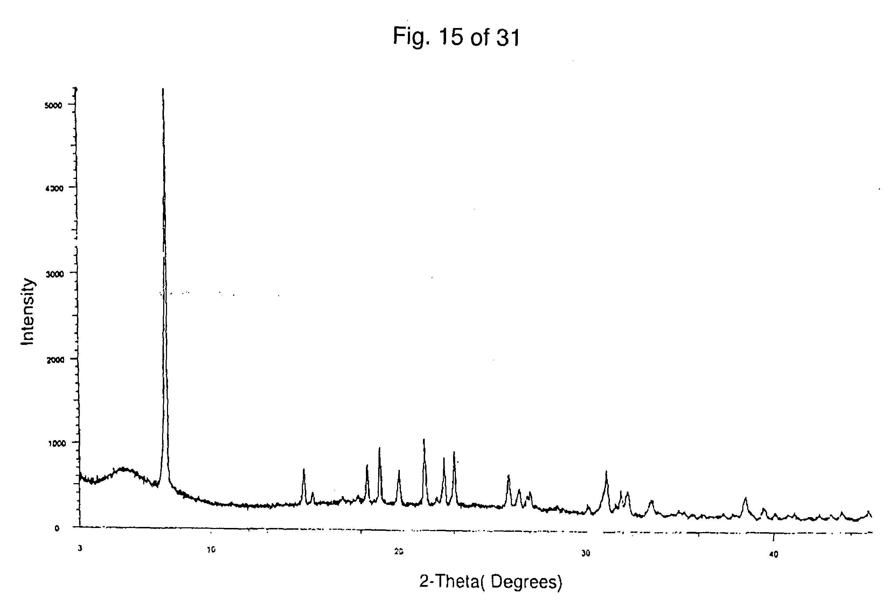

Esomeprazole free base (10 gm) of Formula I and toluene (70 ml) were charged into a clean and dry 4 neck round bottom flask followed by stirring for 10 minutes. Potassium hydroxide (1.623 gm) dissolved in methanol (35 ml) was added slowly at 25-30°C over a period of 10-20 minutes and stirred for 3 hours. The resulting solution was distilled completely at 60 °C under vacuum and toluene (2x60 ml) was charged and distilled to dryness. To the obtained residue, toluene (35 ml) was charged and stirred for 20-30 minutes. The solid was filtered and was washed with toluene (35 ml). The solid obtained was dried at 65°C under vacuum to afford the title compound, characterized by a PXRD pattern substantially in accordance with Fig. 15.

### EXAMPLE 17: PREPARATION OF ESOMEPRAZOLE POTASSIUM CRYSTALLINE FORM P4

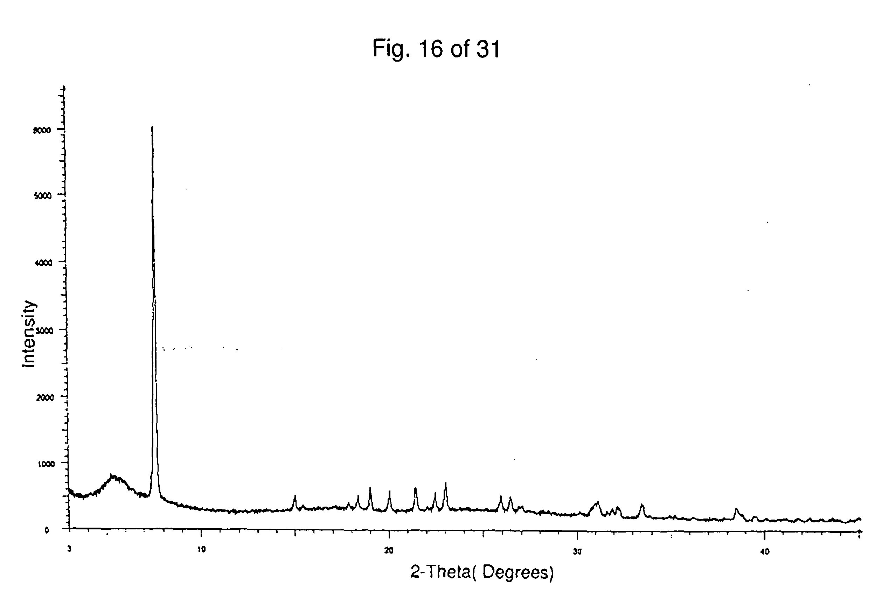

Esomeprazole free base (10 gm) of Formula I and acetonitrile (70 ml) was charged into a clean and dry 4 neck round bottom flask followed by stirring for 10 minutes. Potassium hydroxide (1.623 gm) dissolved in methanol (35 ml) was added slowly at 25-30°C over a period of 10-20 minutes and stirred for 3 hours. The formed solid was filtered and was washed with acetonitrile (35 ml). The solid obtained was dried at 60-70°C under vacuum for 3 hours to afford 3.9 g of the title compound, characterized by a PXRD pattern substantially in accordance with Fig. 16.

### EXAMPLE 18: PREPARATION OF ESOMEPRAZOLE POTASSIUM CRYSTALLINE FORM P5

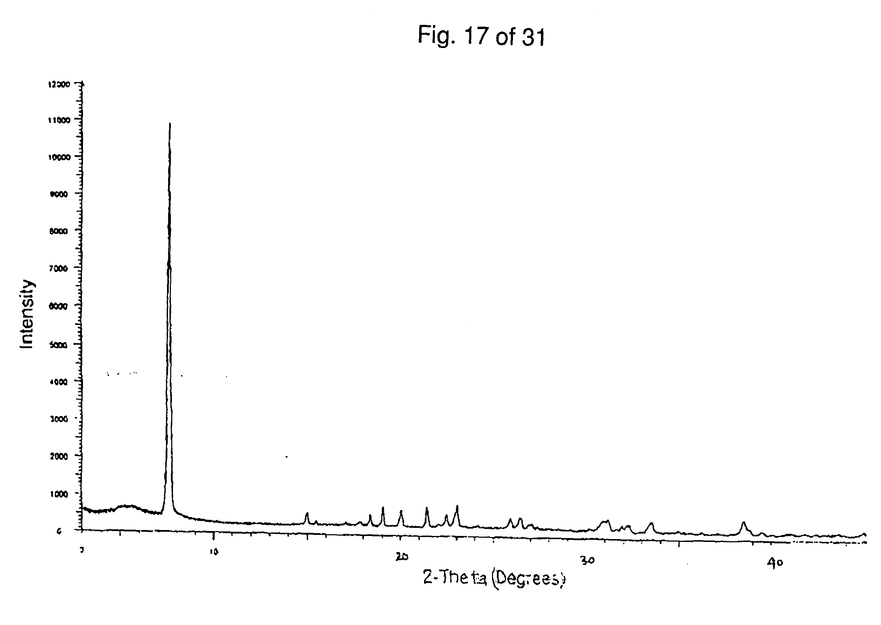

Esomeprazole free base (10 gm) of Formula I and dichloromethane (70 ml) were charged into a clean and dry 4 neck round bottom flask followed by stirring for 10 minutes. Potassium hydroxide (1.623 gm) dissolved in methanol (35 ml) was added slowly at 25-30°C over a period of 10-20 minutes and stirred for 3 hours. The solution obtained was distilled completely at 55°C under vacuum, and dichloromethane (60 ml) was charged and distilled to dryness. To the residue, dichloromethane (60 ml) was added and stirred at 25-30°C for 45-60 minutes. The solid was filtered and was washed with dichloromethane (35 ml). The solid obtained was dried at 65°C under vacuum to afford 3.4 g of the title compound, characterized by a PXRD pattern substantially in accordance with Fig. 17.

### EXAMPLE 19: PREPARATION OF ESOMEPRAZOLE POTASSIUM CRYSTALLINE FORM P6

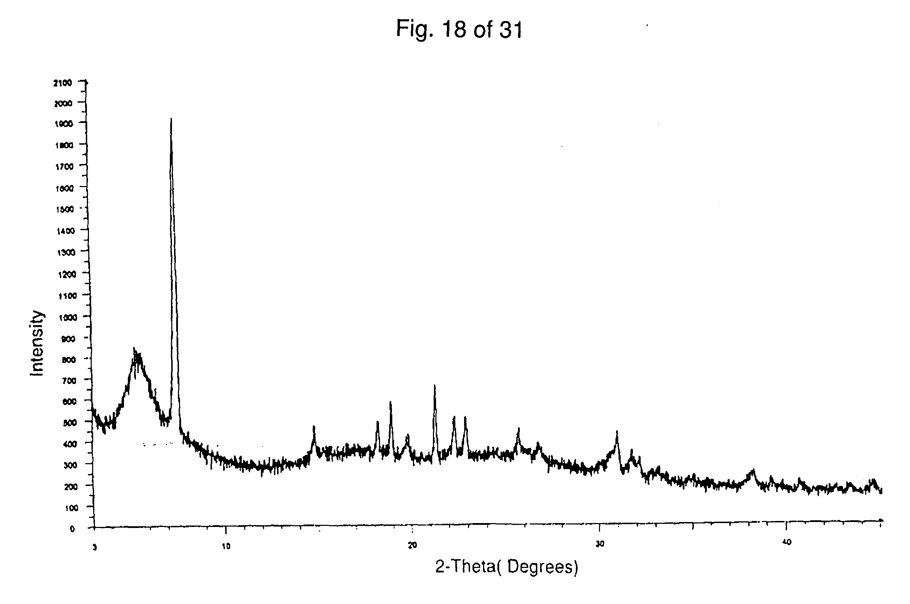

Esomeprazole free base of Formula I and acetone (70 ml) was charged into a clean and dry 4 neck round bottom flask followed by stirring for 10 minutes. A mixture of methanolic potassium hydroxide solution (KOH (1.623 gm) dissolved in methanol (35 ml)) was added slowly at 25-30°C over a period of 10-20 minutes and stirred for 3 hours. Formed solid was filtered and the solid was washed with acetone (35 ml). The solid obtained was dried at 60°C under vacuum for 3 hours to afford 5.3 g of the title compound, characterized by a PXRD pattern substantially in accordance with Fig. 18.

### EXAMPLE 20: PREPARATION OF ESOMEPRAZOLE POTASSIUM CRYSTALLINE FORM P7

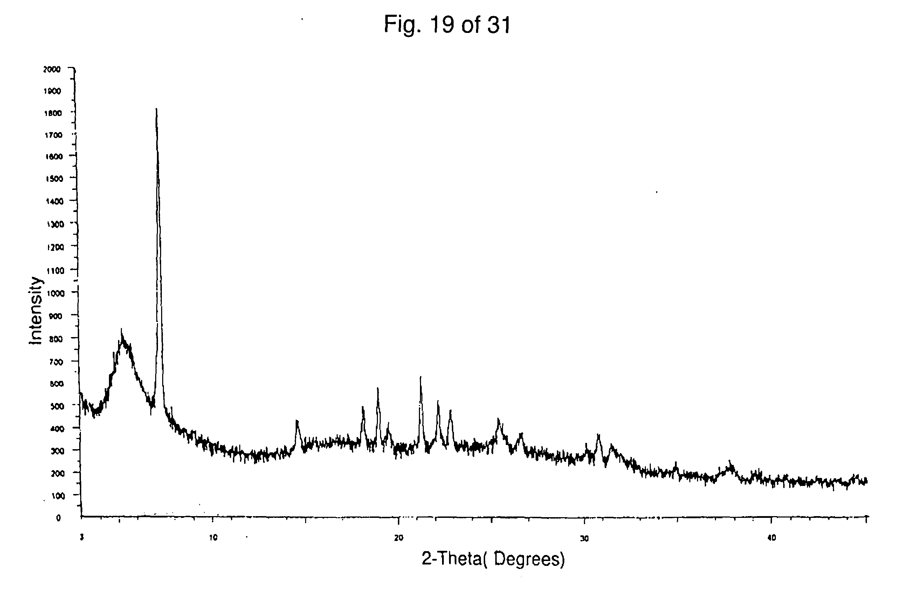

Esomeprazole free base (10 gm) of Formula I and isopropyl alcohol (70 ml) was charged into a clean and dry 4 neck round bottom flask followed by stirring for 10 minutes. A mixture of methanolic potassium hydroxide solution (KOH (1.623 gm) dissolved in methanol (35 ml)) was added slowly at 25-30°C over a period of 10-20 minutes and stirred for 3 hours. The separated solid was filtered and the solid was washed with isopropyl alcohol (35 ml). The solid obtained was dried at 60-70°C under vacuum for about 3 hours to afford 5.3 g of the title compound, characterized by a PXRD pattern substantially in accordance with Fig. 19.

### EXAMPLE 21: PREPARATION OF ESOMEPRAZOLE CALCIUM CRYSTALLINE FORM K1

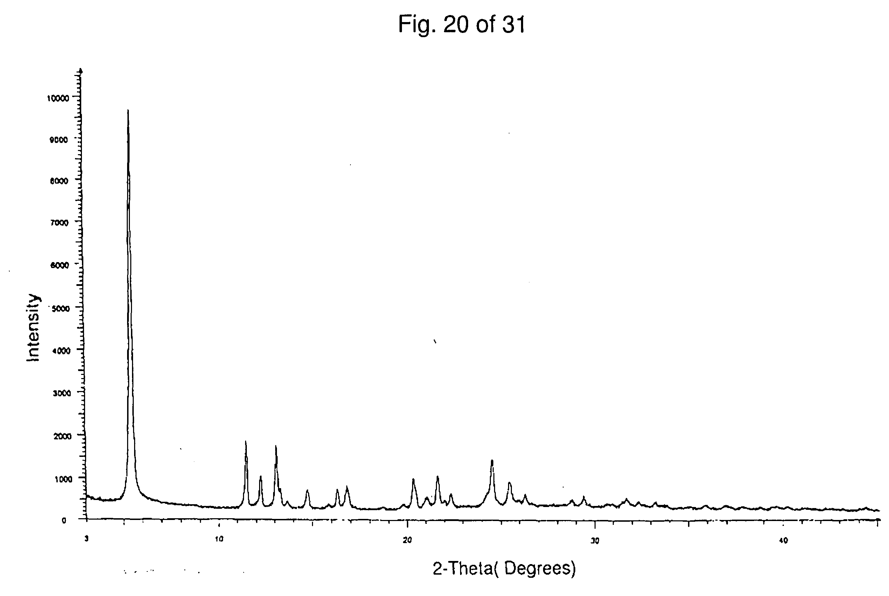

Esomeprazole free base (14.8) of Formula I and water (70 ml) were charged into a clean and dry 4 neck round bottom flask followed by stirring for 10 minutes. Calcium chloride (1.6 gm) dissolved in water (40 ml) was added slowly at 25-30°C over a period of 10-20 minutes and stirred for 3 hours. The formed solid was filtered and was washed with water (35ml). The solid obtained was dried at 60°C under vacuum to afford 7.7 g of the title compound, characterized by a PXRD pattern substantially in accordance with Fig. 20.

### EXAMPLE 22: PREPARATION OF ESOMEPRAZOLE CALCIUM CRYSTALLINE FORM K2

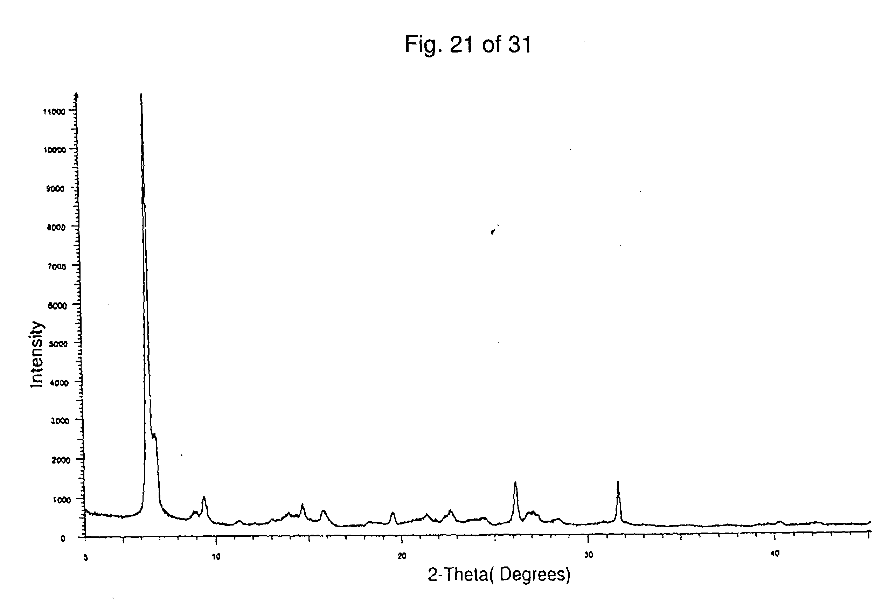

Esomeprazole free base (10 gm) of Formula I, n-butanol (35 ml) and methanol (70 ml) were charged into a clean and dry 4 neck round bottom flask followed by stirring for 10 minutes. Calcium chloride (1.6 gm) dissolved in methanol (40 ml) was added slowly at 25-30°C over a period of 10-20 minutes and was stirred for 3 hours. The unwanted solid was filtered and washed with n-butanol (15 ml). The clear filtrate obtained was distilled completely at 50 °C under vacuum to obtain a semi-solid residue. To the residue, ethyl acetate (80 ml) was added and distilled completely at 60°C under vacuum to afford a residue. To the resultant residue ethyl acetate (35 ml) was charged and stirred at 20-30°C for 30 minutes. The solid was filtered and washed with ethyl acetate (35 ml). The solid obtained was dried at 50 °C under vacuum for 3-4 hours to afford 20 g of the title compound, characterized by a PXRD pattern substantially in accordance with Fig. 21.

### EXAMPLE 23: PREPARATION OF ESOMEPRAZOLE CALCIUM CRYSTALLINE FORM K3

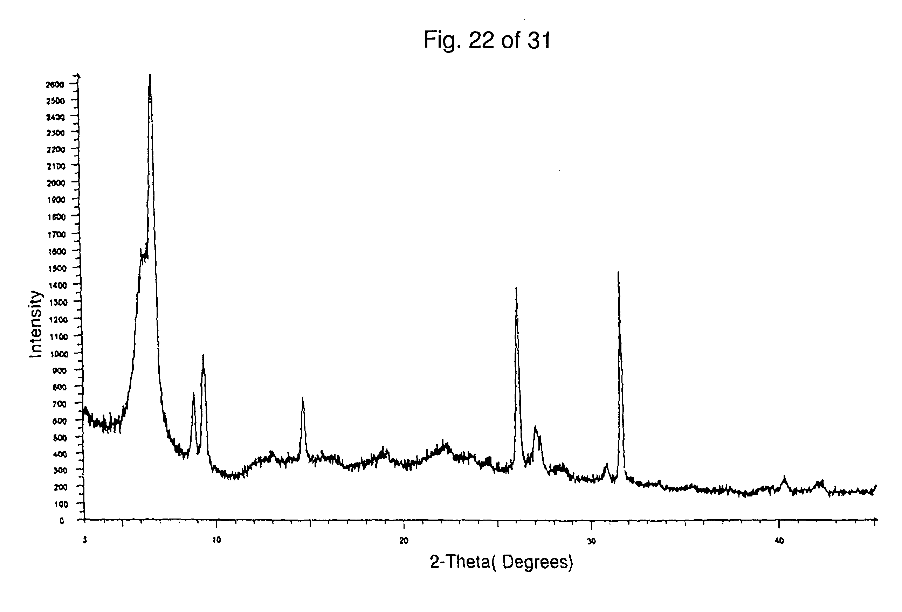

Esomeprazole free base (10 gm) of Formula I, toluene (35 ml) and methanol (70 ml) were charged into a clean and dry 4 neck round bottom flask followed by stirring for 10 minutes. Calcium chloride (1.6 gm) dissolved in methanol (40 ml) was added slowly at 25-30°C over a period of 10-20 minutes and stirred for 3 hours. The unwanted solid was filtered and the solid was washed with toluene (15 ml). The clear filtrate obtained was distilled completely at 50 °C under vacuum to afford a solid residue. To the residue, toluene (35 ml) was charged and distilled completely at 60 °C under vacuum to obtain a residue. To the residue, toluene (60 ml) was charged and stirred at 20-30°C for 30 minutes. The solid was filtered and was washed with toluene (35 ml). The solid obtained was dried at 50°C under vacuum for 2-3 to afford 11.8 g of the title compound, characterized by a PXRD pattern substantially in accordance with Fig. 22.

### EXAMPLE 24: PREPARATION OF ESOMEPRAZOLE CALCIUM CRYSTALLINE FORM K4

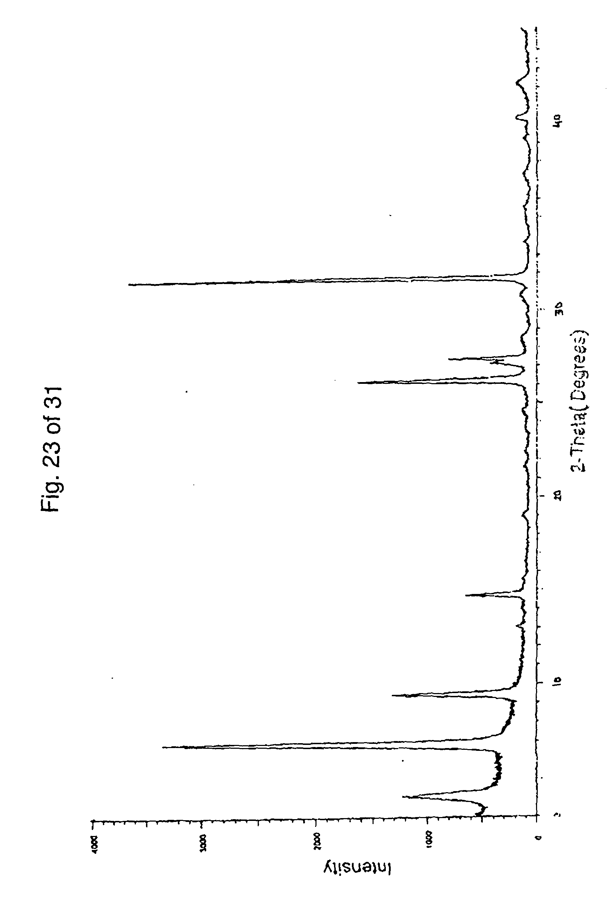

Esomeprazole free base (10 gm) of Formula I and methanol (70 ml) were charged into a clean and dry 4 neck round bottom flask followed by stirring for 10 minutes. Calcium chloride (1.6 gm) dissolved in methanol (40 ml) was added slowly at 25-30°C over a period of 10-20 minutes and stirred for 3 hours. The unwanted solid was filtered and the solid was washed with methanol (35 ml). The filtrate obtained was distilled completely at 60°C under vacuum to afford a residue. To the residue, methanol (50 ml) was charged and distilled completely at 50-60°C under vacuum to obtain a residue. To the residue, acetone (70 ml) was charged and stirred at 30°C for 30 minutes. The solid was filtered and was washed with acetone (35 ml). The solid obtained was dried at 60°C under vacuum for 3-4 hours to afford 15.6 g of the title compound, characterized by a PXRD pattern substantially in accordance with Fig. 23.

### EXAMPLE 25: PREPARATION OF ESOMEPRAZOLE LITHIUM AMORPHOUS FORM

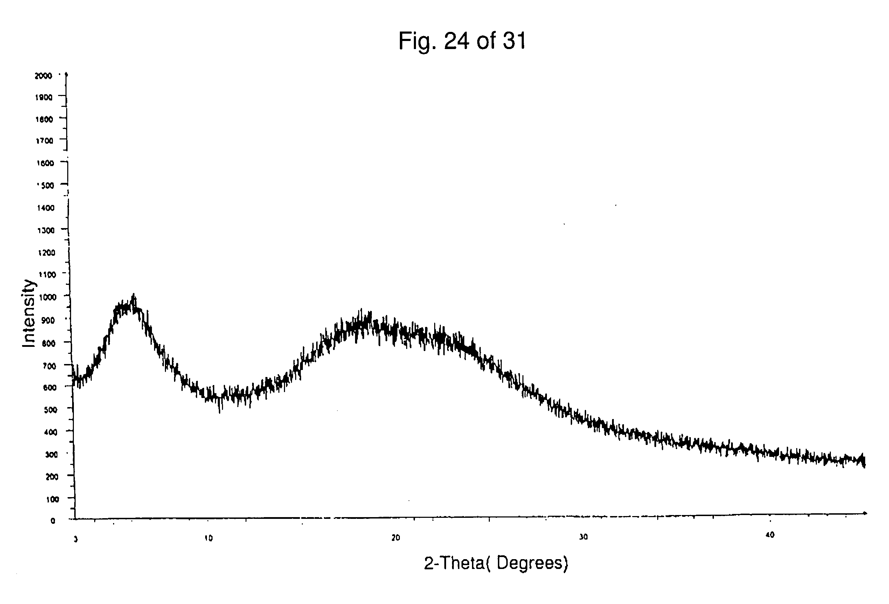

Esomeprazole free base (10 gm) of Formula I and methanol (100 ml) were charged into a clean and dry 4 neck round bottom flask followed by stirring for 10 minutes. Lithium hydroxide (1.2 gm) dissolved in methanol (40 ml) was added slowly at 25-30°C over a period of 10-20 minutes and stirred for 30 minutes. The resultant clear solution was distilled completely at 50 °C under vacuum to obtain 13.5 g of the title compound, characterized by a PXRD pattern substantially in accordance with Fig. 24.

### EXAMPLE 26: PREPARATION OF ESOMEPRAZOLE LITHIUM CRYSTALLINE FORM L1

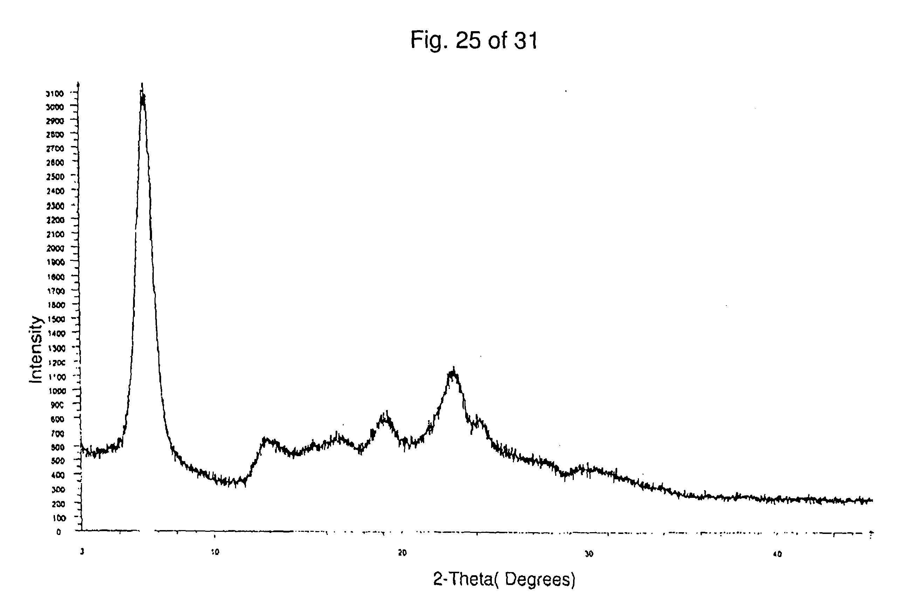

Esomeprazole free base (10 gm) of Formula I and acetone (100 ml) of were charged into a clean and dry 4 neck round bottom flask followed by stirring for 10 minutes. Lithium hydroxide (1.2 gm) dissolved in methanol (30 ml) was added slowly at 25-30°C over a period of 10-20 minutes and stirred for 3 hours. The resultant clear solution was distilled until about 80-90% of the original volume was removed at 50°C under vacuum, acetone (100 ml) was charged, and the mixture was stirred 25-30°C for 30-60 minutes. The solid that formed was filtered and was washed with acetone (20 ml). The solid obtained was dried at 70°C under vacuum for 3-4 hours to afford 4 g of the title compound, characterized by a PXRD pattern substantially in accordance with Fig. 25.

### EXAMPLE 27: PREPARATION OF ESOMEPRAZOLE LITHIUM CRYSTALLINE FORM L2

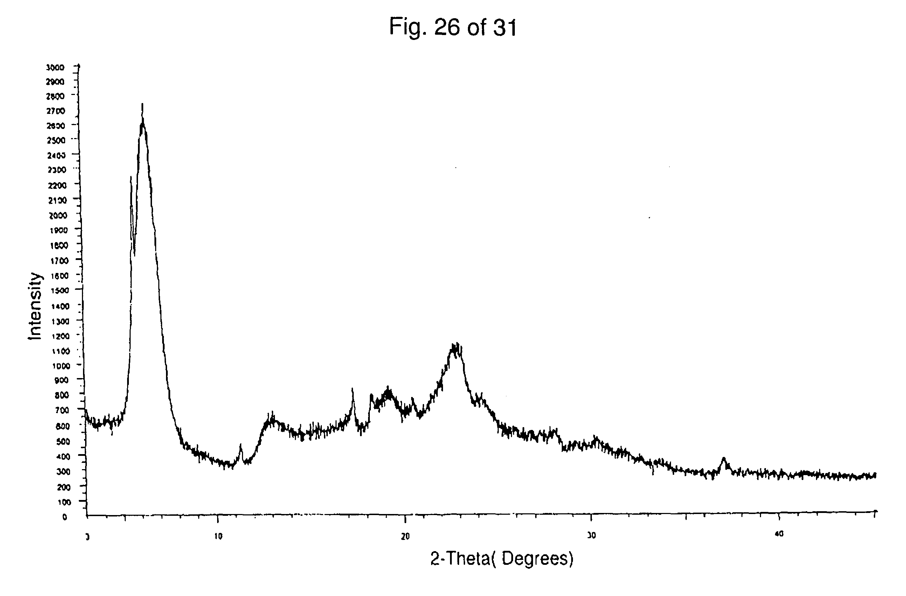

Esomeprazole free base (10 gm) of Formula I and toluene (100 ml) were charged into a clean and dry 4 neck round bottom flask followed by stirring for 10 minutes. Lithium hydroxide (1.2 gm) dissolved in toluene (30 ml) was added slowly at 25-30°C over a period of 10-20 minutes and stirred for 3 hours. The resultant clear solution was distilled until about 80-90% of its original volume was removed at 50°C under vacuum, toluene (100 ml) was added and the mixture was stirred at 25-30°C for 30-60 minutes. The formed solid was filtered and the solid was washed with toluene (20 ml). The solid obtained was dried at 70°C under vacuum for 3-4 hours to afford 5 g of the title compound, characterized by a PXRD pattern substantially in accordance with Fig. 26.

### EXAMPLE 28: PREPARATION OF ESOMEPRAZOLE LITHIUM CRYSTALLINE FORM L3

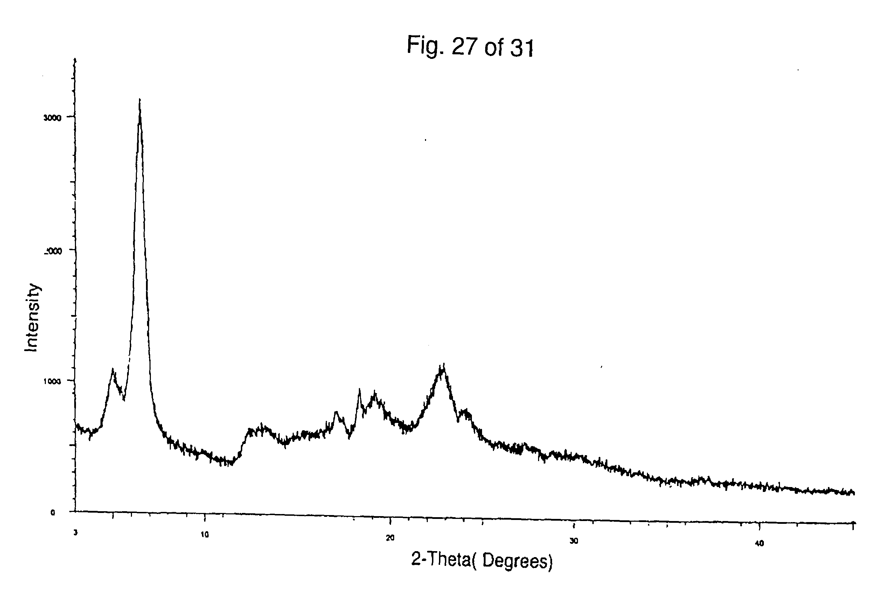

Esomeprazole free base (10 gm) of Formula I and isopropyl alcohol (100 ml) was charged into a clean and dry 4 neck round bottom flask followed by stirring for 10 minutes. Lithium hydroxide (1.2 gm) dissolved in methanol (30 ml) was added slowly at 25-30°C over a period of 10-20 minutes and stirred for 3 hours. The resultant clear solution was distilled until about 80-90% of its original volume was removed at 50°C under vacuum, isopropyl alcohol (100 ml) was charged and the mixture was stirred at 25-30°C for 30-60 minutes. The formed solid was filtered and was washed with isopropyl alcohol (20 ml). The solid obtained was dried at 70°C under vacuum to afford 2.1 g of the title compound, characterized by a PXRD pattern substantially in accordance with Fig. 27.

### EXAMPLE 29: PREPARATION OF ESOMEPRAZOLE LITHIUM CRYSTALLINE FORM L4

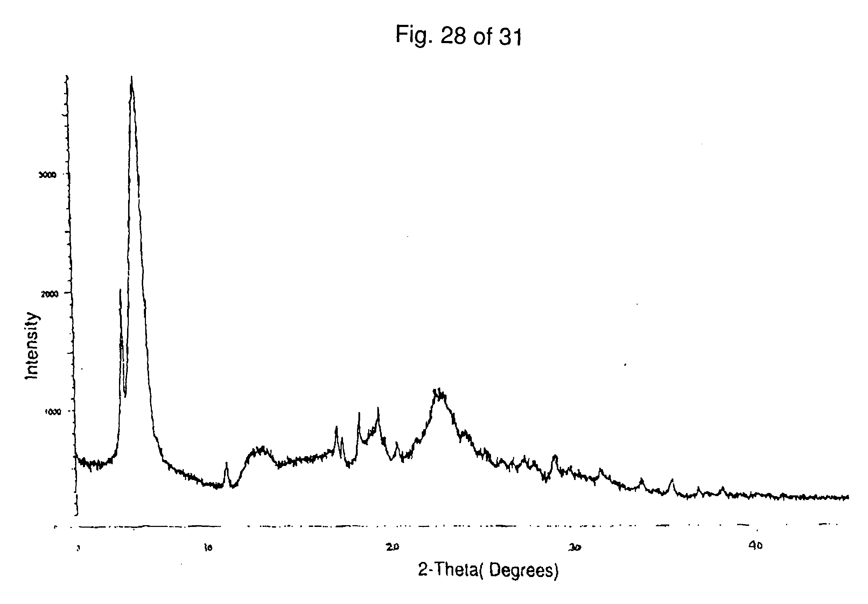

Esomeprazole free base (10 gm) of Formula I and acetonitrile (100 ml) were charged into a clean and dry 4 neck round bottom flask followed by stirring for 10 minutes. Lithium hydroxide (1.2 gm) dissolved in methanol (30 ml) was added slowly at 25-30°C over a period of 10-20 minutes and stirred for 3 hours. The resultant clear solution was distilled to remove about 80-90% of the original volume at 50°C under vacuum, acetonitrile (100 ml) was added and the mixture was stirred at 25-30°C for 30-60 minutes. The formed solid was filtered and was washed with acetonitrile (20 ml). The solid obtained was dried at 70°C under vacuum for 2-3 hours to afford 6.2 g of the title compound, characterized by a PXRD pattern substantially in accordance with Fig. 28.

### EXAMPLE 30: PREPARATION OF ESOMEPRAZOLE LITHIUM CRYSTALLINE FORM L5

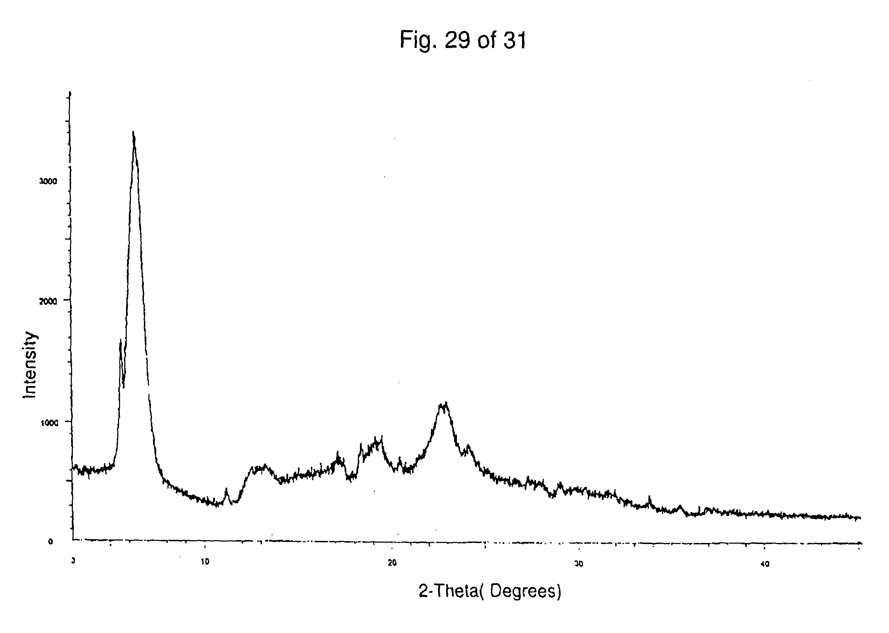

Esomeprazole free base (10 gm) of Formula I and tetrahydrofuran (100 ml) were charged into a clean and dry 4 neck round bottom flask followed by stirring for 10 minutes. Lithium hydroxide (1.2 gm) dissolved in methanol (30 ml) was added slowly at 25-30°C over a period of 10-20 minutes and stirred for 3 hours. The resultant clear solution was distilled to remove about 80-90% of the original volume at 50°C under vacuum, tetrahydrofuran (THF) (100 ml) was added and the mixture was stirred at 25-30°C for 30-60 minutes. The formed solid was filtered and washed with tetrahydrofuran (20 ml). The solid obtained was dried at 70°C under vacuum for 2-3 hours to afford 6 g of the title compound, characterized by a PXRD pattern substantially in accordance with Fig. 29.

### EXAMPLE 31: PREPARATION OF ESOMEPRAZOLE LITHIUM CRYSTALLINE FORM L6

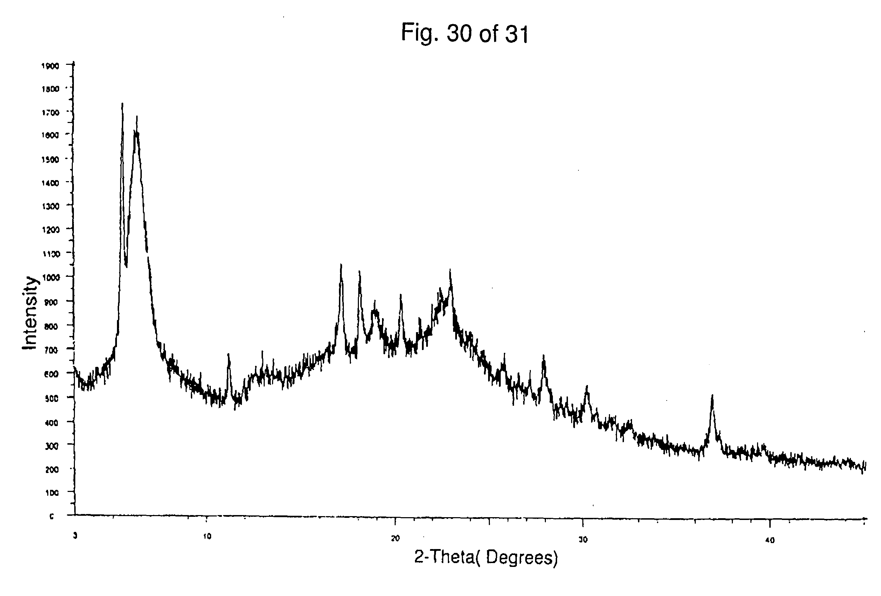

Esomeprazole free base (10 gm) of Formula I and dichloromethane (100 ml) was charged into a clean and dry 4 neck round bottom flask followed by stirring for 10 minutes. Lithium hydroxide (1.2 gm) dissolved in methanol (30 ml) was added slowly at 25-30°C over a period of 10-20 minutes and stirred for 3 hours. The resultant clear solution was distilled to remove about 80-90% of the original volume at 50°C under vacuum, dichloromethane (100 ml) was charged and the mixture was stirred at 25-30°C for 30-60 minutes. The formed solid was filtered and was washed with dichloromethane (20 ml). The solid obtained was dried at 70°C under vacuum for 2-3 hours to afford 7.5 g of the title compound, characterized by a PXRD pattern substantially in accordance with Fig. 30.

### EXAMPLE 32: PREPARATION OF ESOMEPRAZOLE LITHIUM CRYSTALLINE FORM L7

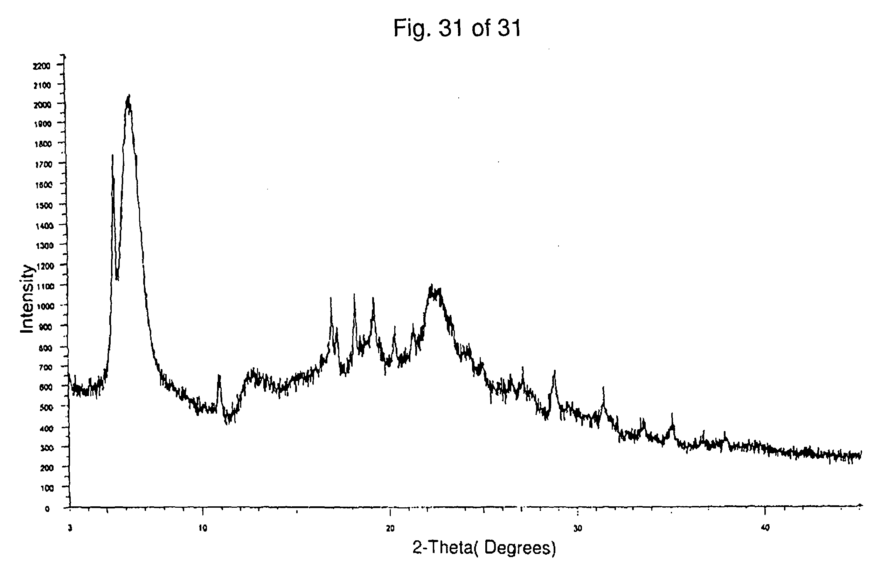

Esomeprazole free base (10 gm) of Formula I and ethyl acetate (100 ml) was charged into a clean and dry 4 neck round bottom flask followed by stirring for 10 minutes. Lithium hydroxide (1.2gm) dissolved in methanol (50 ml) was added slowly at 25-30°C over a period of 10-20 minutes and stirred for 3 hours. The resultant clear solution was distilled to remove about 80-90% of the original volume at 50°C under vacuum, ethyl acetate (100 ml) was added and the mixture was stirred at 25-30°C for 30-60 minutes. The solid was filtered and washed with ethyl acetate (20 ml). The solid obtained was dried at 70°C under vacuum for 2-3 hours to afford 12.8 g of the title compound, characterized by a PXRD pattern substantially in accordance with Fig. 31.

## Claims

1. A process for the preparation of esomeprazole salt or polymorphic form thereof, comprising:
a) providing a solution comprising esomeprazole free base in an organic solvent;
b) adding a base or a salt to the solution ; and
c) recovering the solid to afford the desired salt of esomeprazole or polymorphic form thereof.

2. The process of claim 1, wherein the organic solvent is selected from C1-C4 alcohols; C2-C6 ketones; chlorinated solvents; ethers; hydrocarbon solvents; esters; nitriles; aprotic polar solvents; and mixtures thereof or their combinations with water.

3. The process of claim 1, wherein the base is selected from the group comprising lithium hydroxide, barium hydroxide, potassium hydroxide, calcium hydroxide, copper sulfate, and zinc chloride.

4. A salt of esomeprazole selected from the group consisting of copper, zinc, barium, potassium, calcium and lithium in crystalline form.

5. A salt of esomeprazole selected from the group consisting of copper, zinc, barium and lithium in amorphous form.

6. The polymorphic forms of salts of claim 4 or claim 5 selected from the group consisting of C1, C2, C (amorphous), Z (amorphous), Z1, B (amorphous), B1, B2, B3, B4, B5, B6, P1, P2, P3, P4, P5, P6, P7, K1, K2, K3, K4, L (amorphous), L1, L2, L3, L4, L5, L6, and L7.

7. The polymorphic forms of claim 6 **characterized by** degrees 2-theta ±.0.2 values in PXRD patterns substantially as depicted in Table-1

8. The polymorphic forms of claim 6 **characterized by** PXRD patterns substantially as illustrated by Figures 1-31.
